# EUROPEAN PATENT APPLICATION

(11) **EP 1 371 699 A2**
(43) Date of publication of application: **17.12.2003**
(21) Application number: 03253744.1
(22) Date of filing: 12.06.2003
(51) Int. Cl.: C09D 157/00

(54) **Polymeric nanoparticle and antimicrobial coating formulations**

(30) Priority: 14.06.2002 US 389043 P; 30.09.2002 US 414591 P
(71) Applicant: ROHM AND HAAS COMPANY, Philadelphia, Pennsylvania 19106-2399 (US)
(72) Inventor: Sheppard, Aurelia de la Cuesta, Newtown, Pennsylvania 18940 (US); Lauer, Rosemarie Palmer, Chalfont, Pennsylvania 18914 (US)
(74) Representative: Kent, Venetia Katherine

(57) **Abstract**

A composition and method for controlling the attachment and growth of microbial species on the surface of a coating consisting of adding to the coating formulation polymeric nanoparticles, which have a mean particle diameter of from 1 to 50nm, linked to bioactive molecules are disclosed. The bioactive molecules are linked to the polymeric nanoparticles via either covalent or ionic bonds. The bioactive molecules may either cleave off from the polymeric nanoparticles to become the biologically active molecules or may remain linked to the polymeric nanoparticles, thus making the polymeric / bioactive molecules the biologically active entities.

## Description

The present invention relates to coatings and specifically to compositions which improve the ability of coatings to control the attachment and growth of microbial species on its surface and thereby reduce or altogether eliminate the unsightly build up of mildew and/or various forms of fungi.

For purposes of this invention, microbes are defined as consisting of any microorganism capable of attaching to the surface of a coating. Microbes may be broken down into two categories. The first category consists of fungi which commonly cause the growth of mold on coating surfaces. Some examples of this class include *Aureobasidium, aspergillus, Cladosporium and Penicillium.* The other category is algae, the majority of which fall into the genera *Chroococcus, Chlorococcuum, Gloeocapsa, Protococcus and Trentepohlia.* Bacteria, such as that of the genus *Pseudomonas,* are also included within the definition of "microbes" and are often the initial colonizers in a succession of organisms involved in the formation of microbial caused coating surface blemishes.

Particulate matter attack a coating from the moment the coating is applied. Examples of particulate matter include sand, smoke particles, fungal spores, pollen, building dust, grease, human detritus, fibres, cosmetic powders, oil and carbon black, to name a few. Particles of dirt and dust may carry various species of microbial spores or cells which, over time, may find sufficient sources of nutrients to proliferate into visibly unsightly colonies of fungi or mildew on the surfaces of architectural coatings.

Coating formulations have long included biocidal compounds in an effort to reduce or eliminate microbial growth on the surfaces of these architectural coatings. However, the active life of biocidal compounds in coatings is severely limited by the evaporation or volatilization of these compounds after drying of the coating and unexpected reactions of functional groups within these compounds with other components within the paint formulation.

One potential solution to this problem is offered by U.S. Patent No. 6,194,530 which discloses the use of polymers having antimicrobial properties. The polymers are comprised of macromonomers having at least one alkyl residue that is bonded to a quaternary ammonium group. While linkage with a much larger polymer will slow down the rate of evaporation or volatilization of the bioactive molecule, the large size of the polymer impedes its movement to the surface of the coating, thus minimizing contact between the antimicrobial compound and the target microbes.

What is desired, therefore, is a means to slow down or at least control the rate of release of the antimicrobial compound within the coating formulation after application to the architectural substrate. Further, since the zone of activity for an antimicrobial compound is the surface of a coating, the maximum amount of antimicrobial compound present in the coating formulation must be allowed to migrate to the surface for sustained antimicrobial activity over time.

The present invention comprises polymeric nanoparticles ("PNP") linked to bioactive molecules. Within the context on this application, "linked" means attachment by means of either covalent or ionic bonds. Each PNP has a mean particle diameter of from 1 to 50 nanometers ("nm"), and consists of from 1 to 100%, by weight, of at least one multi-ethylenically unsaturated monomer. Preferably, each PNP may be from 1 to 30 nm and most preferably from 1 to 10 nm in diameter. The term "bioactive molecule" means, within the context of this application, functionalities incorporated into the PNP either during or after polymerization that will cause the PNP to become biologically active, regardless of whether or not the functionality by itself is biologically active. The bioactive functional PNP may then function as the bioactive molecule itself or the bioactive functionality may be released from the PNP through a cleaving mechanism, such as hydrolysis, so that the bioactive functionality performs the function of the bioactive molecule.

The bioactive molecules may comprise amine groups containing one or more amine functionalities. The amines can be selected from the group consisting of primary, secondary, tertiary and quaternary amines and biguanide groups. Substitution of the nitrogen can include substituted or unsubstituted branched or unbranched aliphatic or aromatic hydrocarbon radicals. This includes N-halamine derivatives, such as are disclosed in U.S. Patent No. 6,162,452.

Bioactive molecules may also comprise titanium trialkoxide groups containing one or more titanium trialkoxide functionalities. Another class comprises phosphonium salts containing one or more phosphonium functionalities. Yet another class comprises sulfonium salts containing one or more sulfonium salts. An additional class comprises carbamate derivatives which comprises one or more carbamates groups.

While not being bound to a particular theory, PNPs according to the invention, having specified composition or pendant functional groups, will, due to their very small size and high surface area relative to traditional polymer latexes, tend to enhance the effectiveness of the desired functionalities at the surface of the dried or cured coating. PNPs can be used alone or in conjunction with other latex binders to produce clear and pigmented coatings with improved resistance to microbial attack. PNPs may be used to modify the bioactivity of a coating composition by, for example, incorporating into the PNP composition specifically bioactive compounds or moieties.

In a second embodiment, the formation of PNPs bearing bioactive amine functional groups may be achieved by the introduction of, for instance, primary, secondary, tertiary, or quaternary amines monomers during all or part of the polymerization portion of the PNP preparation. This includes, but is not limited to, nitrogen-containing ethylenically unsaturated monomers and nitrogen-containing vinyl compounds. The level of the nitrogen-bearing monomer may range from 0.5 to 95%, preferably 0.5 to 50%, based on total weight of PNP.

Suitable amine-containing monomers include N,N-dimethylaminoethyl (meth)acrylate, N,N-diethylaminoethyl (meth)acrylate, N-t-butylaminoethyl (meth)acrylate, N,N-dimethylaminopropyl (meth)acrylamide, p-aminostyrene, N,N-cyclohexylallylamine, allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine; monomers having piperidine functionality, such as vinylpiperidines; and monomers having imidazole functionality, which includes vinyl imidazole. Other suitable amine-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like. Other examples are polymerizable quaternary ammonium compounds comprising an antibacterial quaternary ammonium portion and anion of an ethylenically unsaturated acid. These may be exemplified by, for instance, C12 n-alkyl dimethyl ethylbenzyl ammonium salt of an unsaturated sulfonic acid.

Suitable bioactive amphoteric monomers include N-vinylimidazolium sulfonate inner salts and N,N-Dimethyl-N-(3-methacrylamidopropyl)-N-(3-sulfopropyl) ammonium betaine.

PNPs linked to bioactive molecules can be prepared by post-functionalization of a preformed PNP. A monomer bearing a first reactable group, the co-reactive monomer, is incorporated into the PNP during the polymerization portion of the PNP preparation. At some point, a modifying compound bearing the second reactable group, the co-reactive bioactive compound, is combined with the co-reactive monomer to tightly associate the stabilizing group with the PNP. In one example, a carboxylic acid-containing PNP can be post-reacted with a quaternary ammonium compound, such as C10-alkyl dimethyl benzyl ammonium chloride, to yield a PNP with pendant biologically active groups. In another example, an acetoacetate-containing PNP is post-reacted with an polyethoxylated amine, such as t-C₁₂₋₁₄NH(CH₂CH₂O)₁₅H.

A stoichiometric excess of the first complementary reactable group may be present relative to the second complementary group in the co-reactive bioactive compound. In this aspect of the invention the weight of co-reactive bioactive compound for which a stoichiometric equivalent of complementary reactable group is present in the PNP preferably comprises equal to or greater than 1% by weight based on the total weight of polymer, of the PNP composition. A stoichiometric excess of the co-reactive bioactive compound containing the second reactable group may be present relative to the first reactable group. The coreactive bioactive compound containing the second reactable group is present in the coating formulation at the level of from 0.01 to about 10 molar equivalent, based on the combined molar equivalent of the first reactable group in the PNP and other polymer(s) in the paint formulation. More preferably, the modifying compound is present at the level of from 0.1 to 1.0 molar equivalent.

Bioactive monomers and groups introduced via complementary reactable groups may each be present singly or in combination with each other in the PNP composition.

Additional examples of polyethoxylated amines and ammonium salts suitable for post-functionalization of an acid or acetoacetate-containing PNP may include: the quaternary amine salt of Ethomeen ® 0/25 supplied by Akzo Chemicals which is a salt with the formula C₁₈H₃₅(CH₃)N(CH₂CH₂O)ₓH(CH₂CH₂O)_{y}H(I) where x+y = 15 and a molecular weight of about 942; C₁₈H₃₇N(CH₂CH₂O)ₓH(CH₂CH₂O)_{y}H(x+y=15), a tertiary polyethoxylated amine; JEFFAMINE ® ED-600 (supplied by the Texaco Chemical Company).

These ionic or covalent bonds can be formed by the combination of the compounds containing the complementary reactable groups prior to, during, or after the free radical polymerization portion of the PNP preparation. An example of combination of the compounds containing the complementary reactable groups prior to the free radical polymerization portion of the PNP preparation would be the combination of AAEM and Ethomeen® 18/25 at any time prior to their introduction to a reaction apparatus in which the free radical polymerization portion of the PNP preparation occurs. An example of combination of the compounds containing the complementary reactable groups during the free radical polymerization portion of the PNP preparation would be the introduction of Ethomeen® 18/25 to a reaction apparatus in which the free radical polymerization portion of a PNP preparation, in which AAEM is one ethylenically unsaturated monomer, is proceeding. Combination of the compounds containing complementary reactable groups could also proceed at any time after completion of the free radical polymerization portion of the PNP preparation, a procedure hereinafter referred to as post-functionalization of a PNP.

If the PNP is to be used in a coating formulation in combination with another polymer, or polymers, the post-functionalization of the PNP can proceed before or after the combination of the PNP with the other polymer(s). The post-functionalization of the PNP can likewise proceed at any point in the formulation and use of a coating composition, up to and including the point of application to a substrate. Optionally, the polymer(s) with which the PNP is being combined may contain reactable groups complementary to that in the coreactive bioactive compound.

In a third embodiment, the PNP monomer composition contains titanium trialkoxide polymerizable monomers, such as titanium trialkoxide (meth)acrylate. The titanium polymers may become bioactive molecules upon cleavage, such as by hydrolysis, from the PNP after application of the coating. The level of the titanium-bearing monomer may range from 0.5 to 95%, preferably 0.5 to 50%, based on total weight of PNP.

In a fourth embodiment, PNPs are prepared which contain bioactive molecules comprising phosphonium salts. Examples include polymerizable phosphonium salts such as the chloride salt of vinyl benzyl phosphonic acid. The level of the phosphonium salts may range from 0.5 to 95%, preferably 0.5 to 50%, based on the total weight of PNP.

In a fifth embodiment, PNPs are prepared which contain bioactive molecules comprising sulfonium salts. Examples include polymerizable sulfonium salts such as the chloride salt of vinyl benzyl sulfonic acid. The level of the sulfonium salt may range from 0.5 to 95%, preferably 0.5 to 50%, based on the total weight of PNP.

In a sixth embodiment, PNPs are prepared which contain bioactive molecules comprising carbamates groups. Carbamate-functional PNPs may be prepared by reacting PNPs containing vinylamine units with haloformic acid esters. The level of the carbamates may range from 0.5 to 95%, preferably 0.5 to 50%, based on the total weight of the PNP.

The foregoing embodiments of the invention may be practiced independently or they may be combined, as desired, to provide a coating formulation having optimum resistance to microbial fouling.

The polymer PNP-modified coating may comprise additional ingredients, such as thickeners, rheology modifiers, surfactants, pigments, flatting aids, waxes, slip aids, coalescents and/or plasticisers, humectants, tackifiers, wetting aids, antifoaming agents, colorants, and antioxidants, such materials being typical ingredients of water based paints and coatings. The coating composition may also include a post cross-linking agent such as polyaziridine, polyisocyanate, polycarbodiimide, polyepoxide, polyaminoplast, polyalkoxysilane, polyoxazolidine, polyamine and polyvalent metal compounds, to improve chemical and mechanical resistance properties of the cured water based coating once it has been applied to the substrate.

The aqueous composition of the present invention includes an aqueous dispersion of polymeric particles having a mean diameter in the range of from 1 to 50 nanometers (nm), the particles including, as polymerized units, at least one multiethylenically unsaturated monomer and at least one ethylenically unsaturated water soluble monomer. As used herein, the term "dispersion" refers to a physical state of matter that includes at least two distinct phases wherein a first phase is distributed in a second phase, the second phase being a continuous medium. By "aqueous" herein is meant a medium that is from 50 to 100 weight % water, based on the weight of the aqueous medium.

The polymeric particles, referred to herein as polymeric nanoparticles ("PNPs"), are addition polymers, which contain, as polymerized units, at least one multiethylenically unsaturated monomer and at least one ethylenically unsaturated water soluble monomer. Suitable multiethylenically unsaturated monomers useful in the present invention include di-, tri-, tetra-, or higher multifunctional ethylenically unsaturated monomers, such as, for example, divinyl benzene, trivinylbenzene, divinyltoluene, divinylpyridine, divinylnaphthalene divinylxylene, ethyleneglycol di(meth)acrylate, trimethylolpropane tri(meth)acrylate, diethyleneglycol divinyl ether, trivinylcyclohexane, allyl (meth)acrylate, diethyleneglycol di(meth)acrylate, propyleneglycol di(meth)acrylate, 2,2-dimethylpropane-1,3-di(meth)acrylate, 1,3-butylene glycol di(meth)acrylate, 1,4-butanediol di(meth)acrylate, 1,6-hexanediol di(meth)acrylate, tripropylene glycol di(meth)acrylate, triethylene glycol di(meth)acrylate, tetraethylene glycol di(meth)acrylate, polyethylene glycol di(meth)acrylates, such as polyethylene glycol 200 di(meth)acrylate and polyethylene glycol 600 di(meth)acrylate, ethoxylated bisphenol A di(meth)acrylate, poly(butanediol) di(meth)acrylate, pentaerythritol tri(meth)acrylate, trimethylolpropane triethoxy tri(meth)acrylate, glyceryl propoxy tri(meth)acrylate, pentaerythritol tetra(meth)acrylate, dipentaerythritol monohydroxypenta(meth)acrylate, divinyl silane, trivinyl silane, dimethyl divinyl silane, divinyl methyl silane, methyl trivinyl silane, diphenyl divinyl silane, divinyl phenyl silane, trivinyl phenyl silane, divinyl methyl phenyl silane, tetravinyl silane, dimethyl vinyl disiloxane, poly(methyl vinyl siloxane), poly(vinyl hydro siloxane), poly(phenyl vinyl siloxane), and mixtures thereof. The term "(meth)acrylic" includes both acrylic and methacrylic and the term "(meth)acrylate" includes both acrylate and methacrylate. Likewise, the term "(meth)acrylamide" refers to both acrylamide and methacrylamide. "Alkyl" includes straight chain, branched and cyclic alkyl groups.
Typically, the PNPs contain at least 1% by weight based on the weight of the PNPs, of at least one polymerized multiethylenically unsaturated monomer. Up to and including 99.5 weight % polymerized multiethylenically unsaturated monomer, based on the weight of the PNPs, is effectively used in the particles of the present invention. It is preferred that the amount of polymerized multiethylenically unsaturated monomer is from 1% to 80%, more preferably from 1% to 60%, most preferably from 1% to 25%, by weight based on the weight of the PNPs.
The PNPs further contain, as polymerized units, at least one water soluble monomer. By "water soluble monomer" herein is meant a monomer having a solubility in water of at least 7 weight %, preferably at least 9 weight %, and most preferably as least 12 weight %, at a temperature of 25 °C. Data for the water solubility of monomers is found, for example, in "Polymer Handbook" (Second Edition, J. Brandrup, E.H. Immergut, Editors, John Wiley & Sons, New York) and "Merck Index"(Eleventh Edition, Merck & Co, Inc., Rahway, New Jersey). Examples of water soluble monomers include ethylenically unsaturated ionic monomers and ethylenically unsaturated water soluble nonionic monomers. Typically, the amount of the polymerized water soluble monomer is at least 0.5 weight %, based on the weight of the PNPs. Up to and including 99 weight % polymerized water soluble monomer, based on the weight of the PNPs, can be effectively used in the particles of the present invention.

Ethylenically unsaturated ionic monomer, referred to herein as "ionic monomer" is a monomer that is capable of bearing an ionic charge in the aqueous medium in which the PNPs are dispersed. Suitable ionic monomers include, for example, acid-containing monomers, base-containing monomers, amphoteric monomers; quaternized nitrogen-containing monomers, and other monomers that can be subsequently formed into ionic monomers, such as monomers which can be neutralized by an acid-base reaction to form an ionic monomer. Suitable acid groups include carboxylic acid groups and strong acid groups, such as phosphorus containing acids and sulfur containing acids. Suitable base groups include amines. It is preferred that the amount of polymerized ionic monomer based on the weight of the PNPs is in the range from 0.5 to 99 weight %, more preferably in the range of from 1 to 50 weight %, even more preferably from 2 to 40 weight %, and most preferably from 3 to 25 weight %.
Suitable carboxylic acid-containing monomers include carboxylic acid monomers, such as (meth)acrylic acid, acryloxypropionic acid, and crotonic acid; dicarboxylic acid monomers, such as itaconic acid, maleic acid, fumaric acid, and citraconic acid; and monomers which are half esters of dicarboxylic acids, such as monomers containing one carboxylic acid functionality and one C₁₋₆ ester. Preferred are acrylic acid and methacrylic acid. Suitable strong acid monomers include sulfur acid monomers, such as 2-acrylamido-2-methyl propane sulfonic acid, styrene sulfonic acid, vinyl sulfonic acid, sulfoethyl (meth)acrylate, sulfopropyl (meth)acrylate, 2-acrylamido-2-methyl propane sulfinic acid, styrene sulfinic acid, and vinyl sulfinic acid; and phosphorus acid monomers, such as 2-phosphoethyl (meth)acrylate, vinyl phosphoric acid, and vinyl phosphinic acid. Other acid monomers include terminally unsaturated acid containing macromonomers as disclosed in U.S. Patent No. 5,710,227. Phosphorus acid monomers are desirable as they can provide improved adhesion to certain substrates (e.g., metal).
Suitable base-containing monomers include monomers having amine functionality, which includes N,N-dimethylaminoethyl (meth)acrylate, N,N-diethylaminoethyl (meth)acrylate, N-t-butylaminoethyl (meth)acrylate, N,N-dimethylaminopropyl (meth)acrylamide, p-aminostyrene, N,N-cyclohexylallylamine, allylamine, diallylamine, dimethylallylamine, N-ethyldimethylallylamine, crotyl amines, and N-ethylmethallylamine; monomers having pyridine functionality, which includes 2-vinylpyridine and 4-vinylpyridine; monomers having piperidine functionality, such as vinylpiperidines; and monomers having imidazole functionality, which includes vinyl imidazole. Other suitable base-containing monomers include oxazolidinylethyl (meth)acrylate, vinylbenzylamines, vinylphenylamines, substituted diallylamines, 2-morpholinoethyl (meth)acrylate, methacrylamidopropyl trimethyl ammonium chloride, diallyl dimethyl ammonium chloride, 2-trimethyl ammonium ethyl methacrylic chloride, and the like.
Suitable amphoteric monomers include N-vinylimidazolium sulfonate inner salts and N,N-Dimethyl-N-(3-methacrylamidopropyl)-N-(3-sulfopropyl) ammonium betaine.
Suitable functional monomers, in which the functionality is subsequently formed into an acid or base include monomers containing: an epoxide functionality, such as glycidyl (meth)acrylate and allyl glycidyl ether; an anhydride, such as maleic anhydride; an ester; and a halide. Suitable halide-containing functional monomers include vinylaromatic halides and haloalkyl(meth)acrylates. Suitable vinylaromatic halides include vinylbenzyl chloride and vinylbenzyl bromide. Other suitable functional monomers include allyl chloride, allyl bromide, and (meth)acrylic acid chloride. Suitable haloalkyl(meth)acrylates include chloromethyl (meth)acrylate. Suitable functional monomer, in which the functionality is subsequently forming into a nonionic water soluble group, include vinyl acetate. Hydrolysis of the polymerized vinyl acetate provides hydroxyl groups to the PNPs.
Multiethylenically unsaturated monomers that are also water soluble monomers are alternatively used to prepare the PNPs. In such embodiments, these monomers are classified for the purposes of the present invention as both a multiethylenically unsaturated monomer and a water soluble monomer. An example of a water soluble, multiethylenically unsaturated monomer is phosphodi(ethyl methacrylate).

Ethylenically unsaturated water soluble nonionic monomers are referred to herein as "water soluble nonionic monomers". Examples of water soluble nonionic monomers include hydroxyalkyl (meth)acrylates such as hydroxyethyl (meth)acrylate and hydroxypropyl (meth)acrylate; poly(alkylene oxide) esters of (meth)acrylic acid such as poly(ethylene oxide)₂₀ methacrylate and poly(propylene oxide)₁₅₀ acrylate; acrylamide; and methacrylamide. It is preferred that the amount of polymerized water soluble nonionic monomer based on the weight of the PNPs is in the range from 0.5 to 99 weight %, more preferably in the range of from 20 to 90 weight %, even more preferably from 30 to 80 weight %, and most preferably from 40 to 70 weight %. When the PNPs include, as polymerized units, ionic monomer and nonionic monomer, lower levels of polymerized nonionic water soluble monomer are preferred.
The PNPs optionally contain, as polymerized units, one or more third monomers that are not multiethylenically unsaturated monomers and are not water soluble monomers. Suitable third monomers include C₁-C₂₄ alkyl (meth)acrylates, such as methyl (meth)acrylate, ethyl (meth)acrylate, propyl (meth)acrylate, butyl (meth)acrylate, isobutyl (meth)acrylate, hexyl (meth)acrylate, cyclohexyl (meth)acrylate, 2-ethylhexyl (meth)acrylate, octyl (meth)acrylate, decyl (meth)acrylate, dodecyl (meth)acrylate, pentadecyl (meth)acrylate, hexadecyl (meth)acrylate, octadecyl (meth)acrylate, and nonadecyl (meth)acrylate, and mixtures thereof. Other suitable third monomers include vinyl acetate; vinyl versatate; diisobutylene; ureido containing monomers such as N-(ethyleneureidoethyl)-4-pentenamide, N-(ethylenethioureido-ethyl)-10-undecenamide, butyl ethyleneureido-ethyl fumarate, methyl ethyleneureido-ethyl fumarate, benzyl N-(ethyleneureido-ethyl) fumarate, and benzyl N-(ethyleneureido-ethyl) maleamate; vinylaromatic monomers, such as styrene, α-methylstyrene, vinyltoluene, *p*-methylstyrene, ethylvinylbenzene, vinylnaphthalene, vinylxylenes, and nonylphenoxy propenyl polyethoxylated alcohol. The vinylaromatic monomers also include their corresponding substituted counterparts, such as halogenated derivatives, i.e., containing one or more halogen groups, such as fluorine, chlorine or bromine; and nitro, cyano, (C₁-C₁₀)alkoxy, halo(C₁-C₁₀)alkyl, (C₁-C₁₀)alkoxy, carboxy, and the like.

The PNPs have a mean diameter in the range of from 1 to 50 nm, preferably in the range of from 1 to 40 nm, more preferably from 1 to 30 nm, even more preferably from 1 to 25 nm, even further preferably from 1 to 20 nm, and most preferably from 1 to 10 nm. It is further typical that the PNPs have a mean particle diameter of at least 1.5 nm, preferably at least 2 nm. One method of determining the particle sizes (mean particle diameter) of the PNPs is by using standard dynamic light scattering techniques, wherein the correlation functions are converted to hydrodynamic sizes using LaPlace inversion methods, such as CONTIN.
Typically, PNPs including as polymerized units, less than 10 weight % multiethylenically unsaturated monomer, have a glass transition temperature from -90 °C to 170 °C for the composition in the absence of the polymerized multiethylenically unsaturated monomer, as determined by a modulated DSC measurement. PNPs containing as polymerized units, at least 50 weight % multiethylenically unsaturated monomer are considered to have glass transition temperatures of at least 50°C.
The PNPs of the present invention typically have an "apparent weight average molecular weight" in the range of 5,000 to 1,000,000, preferably in the range of 10,000 to 500,000 and more preferably in the range of 15,000 to 100,000. As used herein, "apparent weight average molecular weight" reflects the size of the PNP particles using standard gel permeation chromatography methods, e.g., using THF solvent at 40 °C, 3 Plgel™ Columns (Polymer Labs, Amherst, MA), 100 Angstrom (10 nm), 10³ Angstroms (100 nm), 10⁴ Angstroms (1 micron), 30 cm long, 7.8 mm ID, 1 milliliter per minute, 100 microliter injection volume, calibrated to narrow polystyrene standards using Polymer Labs CALIBRE ™ software.
The PNPs are optionally characterized as having suitable hydrophilicities that allow the PNPs to be dispersed into an aqueous medium. One method to characterize the hydrophilicity of the PNPs is to calculate the Hansch parameter. The Hansch parameter is calculated using a group contribution method. The monomer units forming the polymer are assigned a hydrophobicity contribution and the relative hydrophobicity of the polymer is calculated based on the weight average of the monomers in the polymer. Hansch and Fujita, *J. Amer. Chem. Soc.,* 86, 1616-1626 (1964); H. Kubinyi, *Methods and Principles of Medicinal Chemistry,* Volume 1, R. Mannhold et al., Eds., VCH, Weinheim (1993); C. Hansch and A. Leo, *Substituent Constants for Correlation Analysis in Chemistry and Biology,* Wiley, New York (1979); and C. Hansch, P. Maloney, T. Fujita, and R. Muir, *Nature,* 194. 178-180 (1962).
Values of the hydrophobicity contributions for several monomers are listed in Table 1.

**Table 1**

| Monomer | Hydrophobicity Contribution |
|---|---|
| ethyl acrylate | 2.11 |
| butyl acrylate | 3.19 |
| 2-ethyl hexylacrylate | 5.22 |
| styrene | 4.29 |
| methyl methacrylate | 1.89 |
| ethyl methacrylate | 2.43 |
| butyl methacrylate | 3.51 |
| isobornyl methacrylate | 5.0 |
| butadiene | 4.0 |
| acrylic acid | -2.52 |
| methacrylic acid | -2.2 |
| maleic anhydride | -3.5 |

Preferred PNPs have a Hansch parameter in the range of from -2.5 to 4, preferably from -1 to 3.
The PNPs optionally contain other functional groups, which are provided by the polymerization of monomers containing those groups or precursor groups thereof. Functional groups are optionally attached to the PNPs by reacting the ionic group of the PNP with a suitable compound. For example, PNPs containing carboxylic acid groups are modified to contain pendant hydrophilic groups by reacting carboxylic acid groups with a suitable alcohol, such as a capped polyalkylene oxide. Alternatively, functional groups are affixed to the PNPs through non-radical reactions resulting in the formation of ionic or covalent bonds between a modifying compound containing the groups and complementary reactable groups covalently bound to the PNP as taught in U.S. Patent No. 5,270,380.
The complementary reactable groups in the PNP and modifying compound provide ionic or covalent bonding. Complementary ionic bonding includes acid-base interaction and ion pair bonding of negatively and positively charged atoms. Covalent bonding by complementary reactable groups includes, for example: (a) acetoacetate-aldehyde; (b) acetoacetate-amine; c) amine-aldehyde; (d) amine-anhydride; (e) amine-isocyanate; (f) amine-epoxy; (g) aldehyde-hydrazide; (i) acid-epoxy; (j) acid-carbodiimide; (k) acid-chloro methyl ester; (j) acid-chloro methyl amine; (m) acid-anhydride; (n) acid-aziridine; (o) epoxy-mercaptan; and (p) isocyanate-alcohol. The first or second reactable group in each pair is present either in the PNP or, alternatively, in the modifying compound.
A suitable method to prepare the aqueous composition containing the PNPs dispersed in an aqueous medium includes the steps of preparing a nonaqueous PNP dispersion containing the PNPs dispersed in at least one solvent; and combining the nonaqueous PNP dispersion with an aqueous medium. By "nonaqueous" herein is meant a medium that contains from zero to less than 50 weight % water, based on the weight of the nonaqueous medium. Aqueous compositions containing PNPs that include, as polymerized units, ionic monomers, are optionally partially or completely neutralized prior to, during, or after combining with the aqueous medium.
A suitable polymerization process to prepare the nonaqueous PNP dispersion is free radical solution polymerization of at least one multiethylenically unsaturated monomer, at least one water soluble monomer, and optionally, at least one third monomer. By "solution polymerization" herein is meant free radical addition polymerization in a suitable solvent for the polymer. By "suitable solvent for the polymer" herein is meant that linear random (co)-polymers having substantially similar polymerized monomer units to the PNPs, are soluble in the solvent. Another method for selecting a suitable solvent or mixture of solvents is on the basis of using solubility parameter analysis. According to such methods, the suitability of the solvent is determined by substantially matching the solubility parameters of the PNP and of the solvent, such as the Van Krevelen parameters of delta d, delta p, delta h and delta v. See, for example, Van Krevelen et al., Properties of Polymers. Their Estimation and Correlation with Chemical Structure, Elsevier Scientific Publishing Co., 1976; Olabisi et al., Polymer-Polymer Miscibility, Academic Press, NY, 1979; Coleman et al., Specific Interactions and the Miscibility of Polymer Blends, Technomic, 1991; and A. F. M. Barton, CRC Handbook of Solubility Parameters and Other Cohesion Parameters, 2^{nd} Ed., CRC Press, 1991. Delta d is a measure of dispersive interactions, delta p is a measure of polar interactions, delta h is a measure of hydrogen bonding interactions, and delta v is a measure of both dispersive and polar interactions. Such solubility parameters are alternatively calculated, such as by the group contribution method, or determined experimentally, as is known in the art. A preferred solvent has a delta v parameter within 5 (joule per cubic centimeter)^{1/2}, preferably within 1 (joule per cubic centimeter)^{1/2} of the polymer delta v parameter. Suitable solvents for the polymerization include organic solvents, such as hydrocarbons; alkanes; halohydrocarbons; chlorinated, fluorinated, and brominated hydrocarbons; aromatic hydrocarbons; ethers; ketones; esters; alcohols; and mixtures thereof. Particularly suitable solvents, depending on the composition of the PNP, include dodecane, mesitylene, xylenes, diphenyl ether, gamma-butyrolactone, ethyl acetate, ethyl lactate, propyleneglycol monomethyl ether acetate, supercritical CO2, caprolactone, 2-heptanone, methylisobutyl ketone, acetone, methyl ethyl ketone, diisobutylketone, propyleneglycol monomethyl ether, and alkyl-alcohols, such as isopropanol, decanol, and t-butanol; and supercritical carbon dioxide.
The nonaqueous PNP dispersion is prepared by first charging a solvent, or alternatively, a mixture of solvent and some portion of the monomers, to a reaction vessel. The monomer charge is typically composed of monomers, an initiator, and a chain transfer agent. Typically, initiation temperatures are in the range of from 55 °C to 125 °C, although lower or higher initiator temperatures are possible using suitable low temperature or high temperature initiators known in the art. After the heel charge has reached a temperature sufficient to initiate polymerization, the monomer charge or balance of the monomer charge is added to the reaction vessel. The monomer charge time period is typically in the range of from 15 minutes to 4 hours, although both shorter and longer time periods are envisioned. During the monomer charge, the reaction temperature is typically kept constant, although it is possible to vary the reaction temperature. After completing the monomer mixture addition, additional initiator in solvent can be charged to the reaction and/or the reaction mixture may be held for a time.
Control of PNP particle size and distribution is achieved by one or more of such methods as choice of solvent, choice of initiator, total solids level, initiator level, type and amount of multi-functional monomer, type and amount of ionic monomer, type and amount of chain transfer agent, and reaction conditions.
Initiators useful in the free radical polymerization of the present invention include, for example, one or more of: peroxyesters, alkylhydroperoxides, dialkylperoxides, azoinitiators, persulfates, redox initiators and the like. The amount of the free radical initiator used is typically from 0.05 to 10% by weight, based on the weight of total monomer. Chain transfer reagents are optionally used to control the extent of polymerization of the PNPs useful in the present invention. Suitable chain transfer agents include, for example: alkyl mercaptans, such as dodecyl mercaptan; aromatic hydrocarbons with activated hydrogens, such as toluene; and alkyl halides, such as bromotrichloroethane.
In one method of preparing the aqueous composition of the present invention, at least a portion of the polymerized ionic monomer units of the PNPs are neutralized with at least one neutralizing agent to form an at least partially neutralized nonaqueous PNP dispersion. The polymerized ionic monomer units of the PNPs can be neutralized in a variety of ways. When the polymerized ionic monomer units are acidic, the neutralizing agent is typically a base. Likewise, when the polymerized ionic monomer units are basic, the neutralizing agent is typically an acid. Suitable bases include inorganic and organic bases. Suitable inorganic bases include the full range of the hydroxide, carbonate, bicarbonate, and acetate bases of alkali or alkaline metals. Suitable organic bases include ammonia, primary/secondary/tertiary amines, diamines, and triamines.

Preferred basic neutralizing agents include sodium hydroxide, and ammonium hydroxide. Suitable acids include carboxylic acids, such as acetic acid; dicarboxylic acids; (di)carboxylic/hydroxyl acids; aromatic acids, such as benzoic acid; and a variety of other acids, such as boric, carbonic, citric, iodic, nitrous, nitric, periodic, phosphoric, phosphorous, sulfuric, sulfurous, and hydrochloric acid. None of the foregoing categories of bases and acids, are deemed to be limiting.
The amount of neutralizing agent required to neutralize the nonaqueous PNP dispersion is typically determined on a molar basis of neutralizing agent to polymerized ionic monomer units of the PNPs. Without being bound to a particular theory, the amount of polymerized ionic monomer units (i.e., level of charge) needed to stabilize the PNPs (i.e., maintain particle size during conversion from non-aqueous to aqueous medium) will vary as PNP composition and properties are varied. It is believed that the PNP hydrophobicity, Tg, crosslinking level, and type of counter-ion from the neutralizing agent are important variables. For providing stable aqueous PNP dispersions (i.e., wherein flocculation of the PNPs is minimized), the polymerized ionic monomer units are preferably at least 20%, more preferably at least 50%, even more preferably at least 80%, and most preferably at least 90% neutralized.
Neutralizing the PNPs is alternatively carried out in a variety of ways. In one method, the nonaqueous PNP dispersion is added to a solution containing the neutralizing agent while stirring. Preferably, the neutralizing agent is added as an aqueous solution over time while stirring the nonaqueous PNP dispersion to provide an at least partially neutralized nonaqueous PNP dispersion.
In one method of preparing the aqueous composition containing dispersed PNPs, the at least partially neutralized nonaqueous PNP dispersion is combined with an aqueous medium. The aqueous medium optionally contains the neutralizing agent(s) for neutralizing the PNPs, in which case the nonaqueous PNP dispersion is capable of being simultaneously neutralized and combined with an aqueous medium. The aqueous medium optionally contains surfactants, which are capable of altering the stability of the PNPs, or of altering other properties of the resulting aqueous PNP dispersion, such as its surface tension.
The sequence of admixing the partially neutralized nonaqueous PNP dispersion and the aqueous medium is not critical. Various methods and equipment, which are suitable for mixing are described in *The Chemical Engineer's Handbook, 5*^{*th*} *Edition,* Perry and Chilton, Eds., McGraw-Hill, Ch. 21, 1973. Typically, the aqueous medium is continuously stirred while adding the partially neutralized nonaqueous PNP dispersion to it in order to ensure that the solvent is intimately mixed with the aqueous medium, which minimizes flocculation of the PNPs.
Suitable weight percentages of the PNPs in the aqueous composition, based on total weight of the aqueous composition, are typically from 1 to 90 weight %, more typically from 2 to 75 weight %, even more typically from 4 to 65 weight %, further more typically from 8 to 55 weight %, and most typically from 10 to 45 weight %.
While the preparation of the aqueous composition of the present invention does not require the use of surfactants, and it is typical that the nonaqueous PNP dispersions are substantially free of surfactants, surfactants are optionally included. When present, the amount of surfactants is typically less than 3 weight percent, more typically less than 2 weight percent, even more typically less than 1 weight percent, further typically less than 0.5 weight percent, and even further typically less than 0.2 weight percent, based on total weight of the PNPs.
The aqueous composition is optionally treated to remove at least a portion of the solvent and optionally water, to increase the solids content of the PNPs. Suitable methods to concentrate the PNPs include distillation processes, such as forming azeotropes of water and a suitable solvent; evaporation of solvent or water; drying the aqueous composition by freeze drying or spray drying; solvent extraction techniques; and ultrafiltration techniques. Preferably at least 25 weight %, more preferably at least 50 weight %, even more preferably at least 75 weight %, and most preferably 100 weight % of the solvent is exchanged with water. Removal of the solvent is preferably carried out under conditions that minimize destabilization (i.e., flocculation) of the PNPs.
In an alternative method, the aqueous composition of this invention is prepared by a method including the steps of preparing a nonaqueous PNP dispersion containing the PNPs dispersed in at least one solvent that is both a suitable solvent for the PNPs and is compatible or miscible in water; and combining the nonaqueous PNP dispersion with an aqueous medium. Examples of such suitable solvents for acrylic-containing PNPs, which are also compatible or miscible with water, include isopropanol and ether alcohols (e.g., monobutyl ether of ethylene glycol and monoethyl ether of diethylene glycol). In this method, the PNPs do not require the addition of neutralizing agents to impart particle stability when combined with water.
Alternate embodiments of the aqueous compositions of the present invention have a wide range of PNP content. Typically, the PNP weight fractions range from 0.1 to 99 weight %, more typically from 1 to 90 weight %, even more typically from 2 to 75 weight %, further typically from 5 to 50 weight %, and most typically 10 to 40 weight %, based on the weight of the aqueous composition.

The PNPs may be present in the reaction vessel during the production of larger particles of a second polymer being formed by a second polymerization. This second polymerization is preferably an emulsion polymerization. An example of such a second polymerization in the presence of PNPs is the use of PNPs of the present invention as stabilizers (i.e., dispersants) in emulsion polymerizations according to the methods known for using "high acid" polymeric stabilizers (often referred to as "resin supported emulsion polymerization", such as are disclosed in US 4,845,149 and US 6,020,061).

Among suitable emulsion polymer compositions, any emulsion polymer, copolymer, multi-stage copolymer, interpolymer, core-shell polymer, and the like can be stabilized using the PNPs of the the present invention. While any ethylenically unsaturated monomer may be used, it is preferred that the emulsion polymers which are stabilized are prepared from at least one of (meth)acrylic ester and vinylaromatic monomers.

In carrying out emulsion polymerizations containing the PNP stabilizers of the present invention, all of the typical emulsion polymerization components, conditions, and processes can be used, e.g., any known emulsion polymerization emulsifier (soap) may be present (or even absent), initiators, temperatures, chain transfer agents, reactor types and solids content, and the like.

PNPs can as well act to stabilize larger polymeric polymers when added after the completion of formation said larger polymeric particles. In such cases it may be desirable to add the PNPs to a dispersion of larger polymer particles under conditions favorable to adsorption of the PNPs to the larger particle. The addition of neutralizing agents to polymer dispersions is well known in the art. These neutralizing agents may be used to promote the creation of charge on polymeric particles containing ionizable groups. As an example bases, such as hydroxides (e.g., sodium hydroxide, potassium hydroxide), amines, or ammonia, may be added to a dispersion of polymeric particles containing carboxylic acid groups to de-protonate the acid groups, thus increasing the charge on the particle surface. In such an instance it may be desirable to add PNPs to such a polymer dispersion before any, optionally before all, of the neutralizing agent is added. Likewise, in instances where it is desirable to add other stabilizing agents (e.g. surfactants) to a dispersion of larger polymeric particles, it may desirable to add the PNPs to the dispersion of larger polymeric particles prior to the addition of any, optionally all, of said other stabilizing agents.

As used herein, the following abbreviations shall have the following meanings, unless the context clearly indicates otherwise: C = centigrade; ?m = micron; UV = ultraviolet; rpm = revolutions per minute; nm = nanometer; J = joules; cc = cubic centimeter; g = gram; wt% = weight percent; L = liter; mL = milliliter; MIAK = methyl iso-amyl ketone; MIBK = methyl iso-butyl ketone; PMA = poly(methyl acrylate); CyHMA = cyclohexylmethacrylate; EG = ethylene glycol; DPG = dipropylene glycol; DEA = diethylene glycol ethyl ether acetate;
BzA = benzylacrylate; BzMA = benzyl methacrylate; MAPS = MATS =
(trimethoxylsilyl)propylmethacrylate; PETTA = pentaerythriol tetra/triacetate;
PPG4000DMA = polypropyleneglycol 4000 dimethacrylate; DPEPA =
dipentaerythriol pentaacrylate; TMSMA = trimethylsilyl methacrylate; MOPTSOMS = methacryloxypropylbis(trimethylsiloxy)methylsilane; MOPMDMOS = 3-methacryloxypropylmethyldimethoxysilane; TAT = triallyl-1,3,5-triazine-2,4,6-(1H,3H,5H)-trione; IBOMA = isobornyl methacrylate; PGMEA = propyleneglycol monomethylether acetate; PEGMEMA475 = poly(ethylene glycol methyl ether)methacrylate Mw=475; EUG= eugenol (4-allyl-2-methoxyphenol); and PGDMA = propyleneglycol dimethacrylate.

The term "(meth)acrylic" includes both acrylic and methacrylic and the term "(meth)acrylate" includes both acrylate and methacrylate. Likewise, the term "(meth)acrylamide" refers to both acrylamide and methacrylamide. "Alkyl" includes straight chain, branched and cyclic alkyl groups.

All ranges defined herein are inclusive and combinable.

The solids content of the coating composition may be from about 10% to about 85 % by volume. The viscosity of the aqueous composition may be from 0.05 to 2000 Pa.s (50 cps to 2,000,000 cps), as measured using a Brookfield viscometer; the viscosities appropriate for different end uses and application methods vary considerably.

The coating composition may applied by conventional application methods such as, for example, brush or paint roller, air-atomized spray, air-assisted spray, airless spray, high volume low pressure spray, air-assisted airless spray, and electrostatic spray.

The coating composition on the substrate is typically dried, or allowed to dry, at a temperature from 20°C to 95°C.

The following examples are presented to illustrate further various aspects of the present invention.

### EXAMPLE 1. Preparation of an aqueous PNP dispersion

A dispersion of methyl methacrylate(MMA)/ methacrylic acid(MAA) /trimethylol propane triacrylate(TMPTA) (70/20/10 wt. %) PNPs was prepared via solution polymerization in isopropyl alcohol (IPA) as follows: A 5 liter reactor was fitted with a thermocouple, a temperature controller, a purge gas inlet, a water-cooled reflux condenser with purge gas outlet, a stirrer, and a monomer feed line. To a separate vessel was charged 450 grams of a monomer mixture (A) containing 315 g MMA, 90 g MAA, and 45 g TMPTA. To an additional vessel was charged an initiator mix (B) consisting of 18 g of a 75% solution of t-amyl peroxypivalate in mineral spirits (Triganox 125-C75), and 113 g IPA. A charge of 2330 g IPA was added to the reactor. After sweeping the reactor with nitrogen for approximately 30 minutes, heat was applied to bring the reactor charge to 79°C. When the contents of the reactor reached 79°C, a dual feed of both the monomer mixture (A) and the initiator mix (B) to the reactor. The two mixtures were fed uniformly using feed pumps over 120 minutes. At the end of the monomer and initiator feeds, the batch was held at 79°C for 30 minutes before adding the first of three initiator chasers consisting of 9 g of a 75% solution of t-amyl peroxypivalate in mineral spirits (Triganox 125-C75), and 22.5 g IPA. Additional initiator was added. The batch was then held at 79°C for and additional 2½ hours. At the end of the final hold, the polymerized MAA units of the PNPs were neutralized by addition to the PNP dispersion of a mixture of water with C12-16 benzyl dimethyl ammonium chloride. The neutralized PNP dispersion was transferred to a roto-evaporator and stripped of solvent at ca. 35°C under vacuum. After removing substantially all of the solvent, the PNP dispersion was further diluted with water to ca. 40wt.% PNP in water. Particle size was measured at ~ 5.0 nm.

### Comparative Example 1 - Formulation of Aqueous Exterior Semi-Gloss Architectural Coating

| Composition | |
|---|---|
| Material | Weight (g) |
| *Combine the following materials in a Cowles mixer* | |
| Propylene Glycol | 32.25 |
| Tamol ® 731 (25%) | 14.21 |
| Foamaster VL | 1.04 |
| Ti-Pure ® R-706 | 208.38 |
| Water | 14.41 |

| *Add the following materials with low shear mixing* | |
|---|---|
| RhoplexSG-10M(50%) | 486.95 |
| Texanol ® | 24.29 |
| Foamaster VL | 1.05 |
| Acrysol ® RM-2020 NPR | 25.0 |
| Acrysol ® RM-8W | 5.0 |
| Water | 179.0 |
| Total | 1021.59 |
| ACRYSOL®, RHOPLEX®, and TAMOL® are trademarks of Rohm and Haas Company. TEXANOL® is a trademark of Eastman Chemical Co. Foamaster is a tradename of Cognis Corporation. Ti-Pure® is a trademark of EI DuPont de Nemours. Co. | |

### Example 2 - Formulation of Experimental Aqueous Exterior Semi-Gloss Architectural Coating

| Composition Using PNPs Formed in Example 1 | |
|---|---|
| Material | Weight (g) |
| *Combine the following materials in a Cowles mixer* | |
| Propylene Glycol | 59.91 |
| Tamol ® 731 (25% solids) | 13.67 |
| Foamaster VL | 1.01 |
| Ti-Pure ® R-706 | 200.54 |
| Water | 13.87 |

| *Add the following materials with low shear mixing* | |
|---|---|
| Rhoplex SG-10M (50% solids) | 486.95 |
| Example 1 (25% solids) | 9.74 |
| Texanol ® | 24.29 |
| Foamaster VL | 1.05 |
| Acrysol ® RM-2020 NPR | 25.0 |
| Acrysol ® RM-8W | 5.0 |
| Water | 174.4 |
| Total | 1015.43 |
| ACRYSOL®, RHOPLEX®, and TAMOL® are trademarks of Rohm and Haas Company. TEXANOL® is a trademark of Eastman Chemical Co. Foamaster is a tradename of Cognis Corporation. Ti-Pure® is a trademark of EI DuPont de Nemours. Co. | |

### Coating Evaluation

### Test Methods for Aqueous Exterior Semi-Gloss Architectural Coatings

- Gloss:: A coating composition is drawn down on a Leneta chart (The Leneta Company, Mahwah, N.J.) with a 3-mil Bird film applicator. The sample is dried at 21°C and 50% relative humidity for seven days. 60° gloss is measured with a Micro-TRI-gloss gloss meter (Byk Gardner, Columbia, MD.).
- Mildew Resistance:: Wood panels were painted with the paints described in examples. After air-drying, the panels were placed on exposure at a farm on northeast Florida, in a north-vertical position for 6 months. The mildew ratings are form 0 (completely covered with mildew) to 10 (no mildew growth).

| Sample | Gloss | Mildew Resistance Rating |
|---|---|---|
| Aqueous Coating Composition Formed in Comparative Example 1 | 75 | 0 |
| Aqueous Coating Composition Formed in Example 2 | 75 | 7 |

Dry films of coating of Experimental Coatings 2, containing polymeric nanoparticles, have superior mildew resistance relative to Comparative Coating 1, which does not contain polymeric nanoparticles.

## Claims

1. A bioactive coating composition comprising polymeric nanoparticles linked to at least one bioactive molecule, the said nanoparticles having a mean particle diameter of from 1 to 50nm and consisting of from 1 to 99.0%, by weight, of at least one multi-ethylenically unsaturated monomer.

2. The composition of claim 1 wherein the bioactive molecule is selected from the group consisting of amine compounds, titanium trialkoxide compounds, phosphonium salt compounds, sulfonium compounds and carbamates groups.

3. The composition of claim 2 wherein the amount of bioactive molecule is from 0.5 to 95%, based on the total weigh of the polymeric nanoparticle.

4. The composition of claim 2 wherein the amine compounds are selected from the group consisting of nitrogen containing ethylenically unsaturated monomers, nitrogen containing vinyl compounds and their N-halamine derivatives.

5. The composition of claim 2 wherein the nitrogen containing ethylenically unsaturated monomers and nitrogen-containing vinyl compounds are selected from the group consisting of tertiary amines, quaternary amines, secondary amines, primary amines and monomers having pendant biguanide groups.

6. The composition of claim 2 wherein the nitrogen containing vinyl compounds are selected from the group consisting of amine containing vinyl ethers, vinyl pyridines, alkyl substituted N-vinyl pyridines and polymers of vinylamine and ethyleneimine.

7. The composition of claim 2 wherein the titanium trialkoxide compound is titanium trialkoxide (meth)acrylate.

8. The composition of claim 2 wherein the phosphonium salts are salts of vinyl benzyl phosphonic acid.

9. The composition of claim 2 wherein the sulfonium salt salts are salts the chloride salt of vinyl benzyl sulfonic acid.

10. The composition of claim 2 wherein the carbamate is the reaction product of vinylamine or ethyleneimine containing polymers with haloformic acid esters.

11. The composition of claim 2 wherein the bioactive molecules are incorporated into the polymeric nanoparticles during or after polymerization of the polymeric nanoparticles.

12. A method to control the attachment of microbial species on the surface of a coating comprising adding to the coating the composition of claim 1.
